# EUROPEAN PATENT APPLICATION

(11) **EP 4 488 685 A2**
(43) Date of publication of application: **08.01.2025**
(21) Application number: 24187221.7
(22) Date of filing: 08.07.2024
(51) Int. Cl.: G01N 33/574, G01N 33/68, G06T 7/00, G16H 30/00, G16H 50/00

(54) **SYSTEM COMPRISING ARTIFICIAL INTELLIGENCE INTEGRATED MOLECULAR CYTOLOGY AND RADIOLOGY FOR TRIAGING OF THYROID NODULES**

(30) Priority: 07.07.2023 IN 202341045806
(71) Applicant: Mazumdar Shaw Medical Foundation, Bengaluru, Karnataka 560099 (IN)
(72) Inventor: Suresh, Amritha, 560099 Bommasandra (IN); Mohan, Uma, 560099 Bommasandra (IN); Sunny, Sumsum, 560099 Bommasandra (IN); Kannan, Subramanian, 560099 Bommasandra (IN); Kuriakose, Moni, 560099 Bommasandra (IN); Pillai, Vijay, 560099 Bommasandra (IN)
(74) Representative: Harrison IP Limited

(57) **Abstract**

The disclosed embodiment relates to a composition and system comprising Artificial Intelligence integrated molecular cytology and radiology for triaging of thyroid nodules. More particularly, the composition comprises of biomarkers WGA, Galectin-3, HBME-1 and MAA. The combination of Galectin-3 and WGA expression in cytology-based assay with USG images illustrated a sensitivity of 84% and 96% specificity in delineating benign and malignant nodules. The combination can differentiate cancer and benign nodules of indeterminate nodules with specificity of 93% and sensitivity of 80% using markers and USG. System also uses Artificial Intelligence for triaging of thyroid nodules, including a preprocessed fusion model developed using a fully connected neural network. The preprocessed fusion model achieved an AUC of 0.71, which increased to 0.91 (sensitivity: 84%, specificity: 96%) when combined with post-processing scores. Incorporating clinical parameters of TIRAD Bethesda scores further improved the AUC to 0.98, with sensitivity and specificity of 95% and 96%, respectively.

## Description

This application claims priority from prior Indian provisional specification patent application titled "SYSTEM COMPRISING ARTIFICIAL INTELLIGENCE INTEGRATED MOLECULAR CYTOLOGY AND RADIOLOGY FOR TRIAGING OF THYROID NODULES", application number 2023414045806 filed on 7th July 2023**.** The entire collective teachings thereof being herein incorporated by reference.

### TECHNICAL FIELD

The present disclosure is in the technical field of an *in-vitro* diagnosis of thyroid cancer. More particularly, it is a system comprising artificial intelligence integrated molecular cytology and radiology for triaging of thyroid nodules.

### BACKGROUND OF THE DISCLOSED EMBODIMENT

Thyroid cancer is a disease in which malignant (cancer) cells form in the tissues of the thyroid gland. Thyroid nodules are common but usually are not cancer.

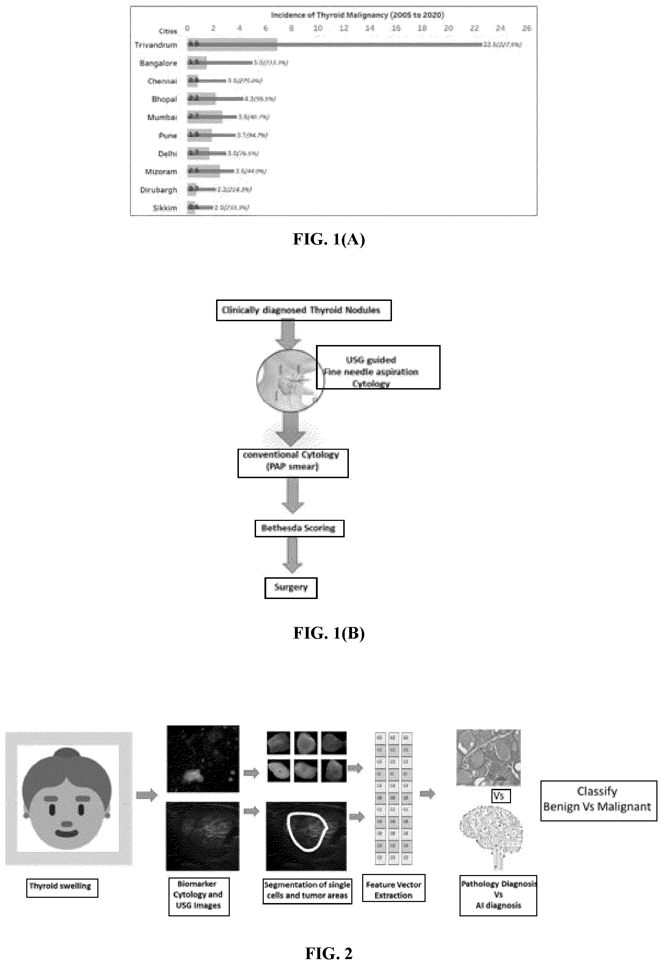

Thyroid malignancies constitute the most common endocrine tumors worldwide with 90% of them involving papillary/follicular thyroid carcinomas. The age-adjusted incidence rate of thyroid cancer in India showed an increasing trend from 7.6 to 14.6/per 100,000 population during the last decade **(****FIG. 1(A)****).**

A similar trend was also observed in developed countries. The standard-of-care diagnostic method for thyroid nodules is Ultrasound-Guided Fine-Needle-Aspiration Cytology (USG-FNAC) and/or Core Needle Biopsy (CNB) integrated with the Bethesda reporting. Patients diagnosed with Bethesda II and V/IV provide accurate diagnoses with benign and malignant pathologies respectively.

However, 30-45% of the diagnosis falls into the indeterminate category (III/IV), with the patients needing diagnostic hemi-thyroidectomies for a definite diagnosis. Thyroid surgery carries the risk of significant complications affecting the patient's quality of life. Diagnostic hemi-thyroidectomies are hence an over-treatment for benign and under-treatment for malignant nodules needing revision surgery, thereby increasing the risk of complications for the patients. A current diagnostic method for thyroid swellings is shown in **FIG. 1(B)****.** Cyto-pathologists and radiologists endocrinologists play a critical role in diagnosing and defining the treatment protocol for thyroid lesions. Although molecular adjuncts have been assessed to improve the accuracy in diagnosis of the indeterminate category, these tests demonstrate variable sensitivity and specificity. Also, the economic considerations and need of advanced technology, preclude its widespread use.

Therefore, there is an urgent need to develop a system comprising artificial intelligence-integrated molecular cytology and radiology for triaging of thyroid nodules.

### SUMMARY OF THE DISCLOSED EMBODIMENT

According to the aspect of the disclosure, a composition for assessing a condition in a biological sample, wherein the composition comprises of biomarkers WGA, Galectin-3, HBME-1 and MAA; a detection agent that binds to the biomarker expression or products; and at least one reagent that allows quantification of biomarkers or biomolecules expression product in a biological sample.

According to an exemplary aspect of the disclosure, the condition is benign or malignancy; wherein biological sample is a thyroid nodule, wherein the biomarkers can differentiate benign from malignant thyroid nodules with sensitivity and specificity, the wherein the biomarkers can predict malignancy or cancer progression.

According to another aspect of the disclosure, the biomarkers can differentiate benign from malignant thyroid nodules wherein the sensitivity and specificity of the biomarkers in differentiating benign from malignant thyroid nodules are WGA: 92.5%/83.8%, MAA: 80%/83.8%, Galectin-3: 72.5%/100% and HBME-1: 90%/70.9% respectively.

According to yet another aspect of the disclosure, the biomarker combination of Gal-3 and WGA expression in cytology-based assay illustrated 85% sensitivity and 96% specificity; wherein the biomarker combination of Galectin-3 and WGA can differentiate cancer and benign nodules of indeterminate nodules with 95% specificity and 80% sensitivity using markers and USG images; wherein the combination of Galectin-3 and WGA markers illustrated an AUC score of 0.96 with 88.3% sensitivity and 90.7% specificity in delineating benign and malignant nodules; wherein the combination of Galectin-3 and WGA markers illustrated an AUC score of 0.78 for indeterminate category and AUC score of 1.0 for category I/II.

According to a further aspect of the disclosure, the detection agent that binds to the biomarker expression products is an antibody or a lectin; wherein the reagent that allows quantification of biomarkers expression product comprises of fluorescent dyes, wherein the fluorescent dyes are DAPI, TRITC, and FITC; wherein the biomolecules in a biological sample are DNA, protein, antibodies.

According to an exemplary aspect of the present disclosure, a method for detection of thyroid cancer in a sample, the method comprising: capturing a set of ultrasonography (USG) images of a thyroid region; capturing a set of molecular cytology images of biological samples of thyroid nodules in the thyroid region; employing a USG-segmentation and classification model to identify a first set of features from the set of USG images, the first set of features characterizing the thyroid nodules in the USG images; employing a molecular cytology-segmentation and classification model to identify a second set of features from the set of molecular cytology images, the second set of pathological features characterizing the thyroid clusters in the molecular cytology images; training a machine learning (ML) model based on a combination of the first set of features and the second set of features; and predicting, based on the ML model, whether a thyroid nodule in the thyroid region is benign or malignant.

According to further aspect of the present disclosure, the method further comprising: developing, using the USG-segmentation and classification model, a USG-AI score for each of the set of USG images; developing, using the molecular cytology-segmentation and classification model, a cytology-AI score for each of the set of molecular cytology images; and providing as inputs to training the ML model, the average of the USG-AI score and cytology-AI score of paired USG and molecular cytology images to generate the post-processing fusion model

According to another aspect of the present disclosure, the ML model is a regression or a non-regression ML model, wherein each of the USG-segmentation and classification model and the molecular cytology-segmentation and classification model (pre-processing fusion and post-processing fusion scores) is provided as an input to a residual convoluted neural network (CNN), the method further comprises: providing as inputs to training the ML model, a clinical diagnosis (Bethesda/TIRAD) of the thyroid region.

According to one more aspect of the present disclosure, the method further comprising: obtaining the biological samples of thyroid nodules from the thyroid region that can be a Fine Needle Aspiration Cytology (FNAC), Core Needle Biopsy (CNB) or a biopsy with adequate sample; applying a set of biomarkers to the biological sample; and capturing images of the stained biological sample to form the set of molecular cytology images.

According to another aspect of the present disclosure, the biomarkers are WGA, Galectin-3, HBME-1 and MAA.

According to further aspect of the present disclosure, an artificial intelligence (AI) system for the detection of thyroid cancer in a sample, the system comprising: a USG-classify model to extract a first set of features from a set of ultrasonography (USG) images captured of a thyroid region, the first set of features characterizing the thyroid nodules in the USG images; a molecular cytology-classify model to extract a second set of features from a set of molecular cytology images captures of biological samples of thyroid nodules in the thyroid region, the second set of features characterizing the thyroid clusters in the molecular cytology images; and a machine learning (ML), preprocessing fusion model trained based a combination of the first set of features and the second set of features, wherein the ML model is thereafter operable to predict whether a thyroid nodule in the thyroid region is benign or malignant (Sensitivity 70%/Specificity 69%).

According to an exemplary aspect of the present disclosure, the USG classify model is operable to predict a USG-AI score for each of the set of USG images, wherein the cytology classify model is operable to predict a cytology-AI score for each of the set of molecular cytology images, and wherein the post-processing ML model is trained with the average of the USG-AI score and cytology-AI score of paired USG and molecular cytology images (Sensitivity: 84%/Specificity: 95%).

According to another aspect of the present disclosure, the ML model is a regression or a non-regression ML model, wherein each of the USG-classify model and the cytology-classify model is a residual convoluted neural network (CNN), is integrated with the pre-processing fusion model, wherein the regression ML model is trained with a clinical diagnosis (Bethesda/TIRAD) of the thyroid region to develop a Thyroid Tumor Score (TTS) (Sensitivity/Specificity >94%).

According to further aspect of the present disclosure, the AI system further comprises: a first segmentation model to provide a first segmentation of the set of USG images, wherein the USG-classify model is operable based on the first segmentation; and a second segmentation model to provide a second segmentation of the set of molecular cytology images, wherein the molecular cytology-classify model is operable based on the second segmentation.

According to yet another aspect of the present disclosure the first segmentation model, and the second segmentation model are U-Nets (IoU>0.80).

Several aspects of the disclosed embodiment are described below with reference to examples for illustration. However, one skilled in the relevant art will recognize that the disclosed embodiment can be practiced without one or more of the specific details or with other methods, components, materials and so forth. In other instances, well-known structures, materials, or operations are not shown in detail to avoid obscuring the features of the disclosed embodiment. Furthermore, the features/aspects described can be practiced in various combinations, though only some of the combinations are described herein for conciseness.

### BRIEF DESCRIPTION OF THE DRAWINGS

Example embodiments of the present disclosure will be described with reference to the accompanying drawings briefly described below.
**FIG. 1(A)** illustrates the incidence of thyroid malignancy: graph depicting 100-200% increase in incidence of thyroid malignancy in India, according to an aspect of the disclosed embodiment.
**FIG. 1(B)** illustrates the current diagnostic method for thyroid swellings, according to an aspect of the disclosed embodiment.
**FIG. 2** illustrates a system for the classification of thyroid nodules as benign or malignant, according to an aspect of the disclosed embodiment.
**FIG. 3(A)** illustrates that Galectin differentiated indeterminate category in meta-analysis. Forest plot analysis of Galectin-3 showed a pooled sensitivity of 76%, according to an aspect of the disclosed embodiment.
**FIG. 3(B)** illustrates that Galectin differentiated indeterminate into benign and malignant in cytology. Forest plot analysis of Galectin-3 showed pooled specificity of 86%, according to an aspect of the disclosed embodiment.
**FIG. 3(C)** illustrates the Galectin-3 association with malignant thyroid nodules. Forest plot showing Diagnostic Odds Ratio (DOR) of Galectin-3 (14.46) in delineating indeterminate category of thyroid nodules in FNAC samples, according to an aspect of the disclosed embodiment.
**FIG. 3(D)** illustrates the HBME-1 association with malignant thyroid nodules- Forest plot showing DOR (11.19) of marker HBME-1 in delineating indeterminate category of thyroid nodules in FNAC sample, according to an aspect of the disclosed embodiment.
**FIG. 4(A)** illustrates the immunohistochemistry (IHC) marker profiles significantly increased in malignant thyroid samples (Papillary Thyroid Cancer: PTC and Follicular Thyroid Cancer: FTC) compared to benign (objective magnification in 10X and 40X), according to an aspect of the disclosed embodiment.
**FIG. 4(B)** illustrates the WGA and Galectin marker staining in malignant tissues is higher as compared to benign tissues. Further, WGA level is high in follicular cancer compared to Galectin 3 and the levels in the benign cohort, according to an aspect of the disclosed embodiment.
**FIG.s 4(C)** and **4(D)** illustrate that the combination of WGA and Galectin 3 differentiated benign and malignant thyroid nodules (ROC AUC: 0.92) compared to single markers, according to an aspect of the disclosed embodiment.
**FIG. 5(A)** illustrates the comparative WGA immunohistochemistry (IHC) scores of benign, follicular adenoma, follicular thyroid carcinoma, papillary thyroid carcinoma. WGA delineated carcinoma from benign nodules including follicular adenoma, according to an aspect of the disclosed embodiment.
**FIG. 5(B)** illustrates the comparative Galectin-3 expression (IHC) of benign, follicular adenoma, follicular thyroid carcinoma, papillary thyroid carcinoma. Galectin delineated carcinoma from benign nodules and adenoma could not be delineated, according to an aspect of the present disclosure.
**FIG. 5(C)** illustrates Galectin-3 and WGA combination delineated the benign and malignant nodules of indeterminate (Bethesda III-IV) or TIRAD (T3, T4) category, (ROC AUC: 0.87), according to an aspect of the disclosed embodiment.
**FIG. 6** illustrates the flow chart for analyzing molecular markers in FNAC samples, according to an aspect of the disclosed embodiment.
**FIG. 7(A)** illustrates the marker manual evaluation in cytology, according to an aspect of the disclosed embodiment.
**FIG. 7(B)** illustrates representative images of multiplex cytology validation of Galectin-3 and WGA, according to an aspect of the disclosed embodiment.
**FIG. 8** illustrates the segmentation plan for thyroid molecular cytology images using U-Net, according to an aspect of the disclosed embodiment.
**FIG. 9(A)** illustrates the training and validation IoU score of segmentation of thyroid nodules using Atrous UNet, according to an aspect of the disclosed embodiment.
**FIG. 9(B)** illustrates the segmentation result of the predicted mask using the trained Atrous U-Net model, according to an aspect of the disclosed embodiment. The testing image, the testing label and the prediction of the test image are indicated.
**FIG. 9(C)** illustrates training and validation F1 score of thyroid nodules for classification of the USG images of TIRAD 1/2 from TIRAD 4/5 using ResNet modified model, according to an aspect of the disclosed embodiment.
**FIG. 9(D)** illustrates training and validation F 1 score of the classification of the thyroid nodules, Bethesda-wise using the USG images (Bethesda I/II from Bethesda V/VI) using the ResNet model, according to an aspect of the disclosed embodiment.
**FIG. 10** illustrates the workflow for USG image analysis, according to an aspect of the disclosed embodiment.
**FIG. 11(A)** illustrates the training and validation IoU score of segmentation of thyroid cluster cells of multiplex molecular cytology (WGA, Galectin-3, and DAPI) using UNet, according to an aspect of the disclosed embodiment.
**FIG. 11(B)** illustrates the segmentation result of the predicted mask using trained U-Net, according to an aspect of the disclosed embodiment. The testing image and label, along with the prediction and the overlay (ground truth-prediction) are provided.
**FIG. 11(C)** illustrates the training and validation F1 score (Y-axis) against the number of epochs (X axis) for the classification of the benign and malignant cluster cells as per histological diagnosis using a modified ResNet model, according to an aspect of the disclosed embodiment.
**FIG. 12** illustrates pipeline for development of the Thyroid Tumor Score (TTS) combining molecular cytology and USG images, according to an aspect of the disclosed embodiment.
**FIG. 13 (A)** illustrates training and validation accuracy of the pre-processed fusion model developed using the benign/malignant cluster cells and USG images after feature extraction using a ResNet model, according to an aspect of the disclosed embodiment.
**FIG. 13(B)** illustrates ROC analysis of post-processing fusion model for the classification of benign and malignant nodules combining Cytology-AI and USG-AI scores; Sensitivity: 84% and specificity: 96% (AUC: 0.91), according to an aspect of the disclosed embodiment.
**FIG. 13(C)** illustrates ROC analysis of combining post-processing fusion model with the Bethesda and TIRAD categories for the classification of benign and malignant; Sensitivity: 95% and specificity: 96% (AUC: 0.98), according to an aspect of the disclosed embodiment.
**FIG. 14** illustrates the final pipeline for classification of benign and malignant thyroid nodule by concatenating the clinical diagnosis (Bethesda/TIRAD), preprocessing fusion model and post-processing fusion model, according to an aspect of the disclosed embodiment.
**FIG. 15** is a block diagram illustrating the details of a digital processing system in which various aspects of the present invention are operative by execution of appropriate execution modules, firmware, or hardware components, according to an aspect of the disclosed embodiment.

In the drawings, like reference numbers generally indicate identical, functionally similar, and/or structurally similar elements. The drawing in which an element first appears is indicated by the leftmost digit(s) in the corresponding reference number.

### DETAILED DESCRIPTION OF THE DISCLOSURE

It is to be understood that the present disclosure is not limited in its application to the details of construction and the arrangement of components set forth in the following description or illustrated in the drawings. The present disclosure is capable of other embodiments and of being practiced or of being carried out in various ways. Also, it is to be understood that the phraseology and terminology used herein is for the purpose of description and should not be regarded as limiting.

The use of "including", "comprising" or "having" and variations thereof herein is meant to encompass the items listed thereafter and equivalents thereof as well as additional items. The terms "a" and "an" herein do not denote a limitation of quantity, but rather denote the presence of at least one of the referenced items. Further, the use of terms "first", "second", and "third", and the like, herein do not denote any order, quantity, or importance, but rather are used to distinguish one element from another. Further, the terms "comprising," "including," "having," and the like, as used with respect to embodiments of the present disclosure, are synonymous. As used herein, the terms "agent," "component," or "ingredient" are used interchangeably and refer to a particular item that includes one or more chemical compounds (e.g., a food item from one or more plants that comprise one or more naturally occurring chemical compounds).

The term "component" or "ingredient" as used herein, refers to a particular item that includes one or more chemical compounds, i.e., edible compounds sourced from plants, fungi or algae that comprise one or more naturally occurring chemical compounds.

Alternative embodiments of the present disclosure and their equivalents may be devised without parting from the spirit or scope of the present disclosure. It should be noted that any discussion herein regarding "one embodiment", "an embodiment", "an exemplary embodiment", and the like indicate that the embodiment described may include a particular feature, structure, or characteristic and that such particular feature, structure, or characteristic may not necessarily be included in every embodiment. In addition, references to the foregoing do not necessarily comprise a reference to the same embodiment. Finally, irrespective of whether it is explicitly described, one of ordinary skill in the art would readily appreciate that each of the particular features, structures, or characteristics of the given embodiments may be utilized in connection or combination with those of any other embodiment discussed herein.

As used herein, the singular forms "a", "an", and "the" include both singular and plural referents unless the context clearly dictates otherwise. By way of example, "a dosage" refers to one or more than one dosage.

The terms "comprising", "comprises" and "comprised of" as used herein are synonymous with "including", "includes" or "containing", "contains", and are inclusive or open-ended and do not exclude additional, non-recited members, elements or method steps.

All documents cited in the present specification are hereby incorporated by reference in their totality. In particular, the teachings of all documents herein specifically referred to are incorporated by reference.

Example embodiments of the disclosed embodiments are described with reference to the accompanying figures.

In the drawings, like reference numbers generally indicate identical, functionally similar, and/or structurally similar elements. The drawing in which an element first appears is indicated by the leftmost digit(s) in the corresponding reference number.

### 1. DEFINITIONS

The term "Thyroid cancer" refer to a disease where cancer cells form in the thyroid gland, while thyroid nodules are typically non-cancerous.
The "(USG-FNAC)" is expanded Ultrasound-Guided Fine-Needle-Aspiration Cytology.

The term "Hemi-thyroidectomies" refers to removal entire thyroid lobe and isthmus on one side, with or without central neck lymph node removal.

The term "Thyroid nodule" refer to an abnormal growth of cells in the thyroid gland, which can be benign, malignant (thyroid cancer), or rarely, caused by other cancers or infections. It can also include fluid-filled cysts or a group of small nodules.

The term "Artificial intelligence" refer to the simulation of human intelligence processes by machines, particularly computer systems. It encompasses applications such as expert systems, natural language processing, speech recognition, and machine vision.

The term "Molecular markers" refer to a protein, DNA fragments associated with specific locations in the genome, used in molecular biology and biotechnology to identify particular DNA sequences within an unknown pool of DNA.

The term "USG IMAGE (Ultrasonography)" refer a noninvasive diagnostic imaging method using ultrasound.

The term "Wheat-Germ-Agglutinin (WGA)" refer to a lectin, a type of protein that binds specifically to certain sugar molecules. It is extracted from wheat germ and is commonly used in biochemical research.

The term "Maackia-Amurensis-Agglutinin (MAA)" refer to a type of lectin derived from the bark of the Maackia-Amurensis tree, native to east Asia. Maa has specific binding affinity for sialic acid-containing glycoconjugates, particularly those with sialylated glycans. MAA is used in biochemical and medical research to study cell surface glycoconjugates, glycoproteins, and the role of sialic acids in cell interactions, signaling, and disease processes. It is also utilized in the purification and characterization of glycoproteins and for histological staining to detect specific sialylated structure in tissues.

The term "Galectine (or Gal-3)" refer to a type of protein belonging to the galectin family, which binds to beta-galectin family, which binds to beta-galactosidase sugars. Galectin-3 plays a role in various biological processes, including cell adhesion, cell growth, apoptosis (programmed cell death), immune response, inflammation, and cancer progression. It is found both inside and outside cells and can be involved in signaling pathway that affect cell behavior.

The term "Hector Battifora Mesothelial-1 (HBME-1)" refer to an immunohistochemical marker commonly used in pathology to aid in the diagnosis of certain types of cancers, particularly thyroid cancer and mesothelioma. HBME-1 is often used to distinguish between benign and malignant thyroid lesions. It is particularly useful in identifying follicular and papillary thyroid carcinomas.

The term "Mutation profile" represent the context-dependent tendencies of mutational processes within the genome, independent of selection biases, achieved by excluding recurring mutations at the same genomic site.
The term "Radiology" refers to a branch of medicine focused on using radiant energy for the diagnosis and treatment of diseases. It encompasses diagnostic radiology and interventional radiology, and practitioners in this field are called radiologists.

The term "Bovine Serum Albumin" refer to a serum albumin protein derived from cows, commonly used as a protein concentration standard in laboratory experiments.

The term "DAPI (4',6-diamidino-2-phenylindole) channel" refers to a blue-fluorescent DNA stain that significantly enhances fluorescence upon binding to AT regions of double-stranded DNA.

The term "TRITC (Tetramethylrhodamine-isothiocyanate) channel" refer to a fluorescent compound with an excitation peak at 544 nm and an emission peak at 570 nm.

The term "FITC (Fluorescein isothiocyanate) channel" refer to fluorescent reagents widely used in biological research due to their high absorptivity, excellent fluorescence quantum yield, and good water solubility.

The term "Semantic segmentation" refer to a deep learning algorithm that assigns a label or category to each pixel in an image.
The term "Convolution-Neural-Networks (CNN)" refer to a type of neural network architecture commonly employed in Computer Vision tasks.

The term "Intersection over Union (IoU)" refer an evaluation metric used to measure the accuracy of an object detector on a specific dataset.

The term "Cytospin" refers to a laboratory technique used to concentrate cells from a fluid sample (like blood, cerebrospinal fluid or other body fluids) onto a slide for microscopic examination.

The term "PBS" stands for phosphate-buffered saline. It is a buffer solution commonly used in biological research.

The term "Pap pen" refers to a tool, used in histology and immunohistochemistry to draw hydrophobic barriers on glass slides.

The term "Triton X-100" refers to a nonionic surfactant and detergent widely used in biochemistry and molecular biology.

The term "VGG" refers to a model that shows simplicity and effectiveness in image classification and object detection tasks.

The term "ResNet" refers to a residual learning, which helps mitigate the vanishing gradient problem encountered in very deep networks.

The term "Atrous U-Net" refers to a variant of the U-net architecture that incorporates atrous convolutions.

The term "ROC (Receiver Operating Characteristic) analysis" refers to a technique used to evaluate the performance of binary classification models.

### 2. GENERAL FLOW

The present disclosure relates to the system comprising artificial intelligence integrated molecular cytology and radiology for triaging of thyroid nodules.

Thyroid cancer is a disease in which malignant (cancer) cells form in the tissues of the thyroid gland. Thyroid nodules are common but usually are not cancer.

In the present disclosure the method comprises of integration of multiplexed molecular cytopathology and USG images of subjects histologically diagnosed with benign and malignant nodules (Bethesda II and V/VI) for developing Thyroid Tumor Score (TTS). Features were extracted from the images to develop AI/ML model.
The patient with thyroid swelling will be subjected USG-guided FNAC. The biomarker-based cytology images will be subjected to segmentation of thyroid cells and feature extraction of cells carried out artificial intelligence. The USG images features will be combined with molecular cytopathology data.

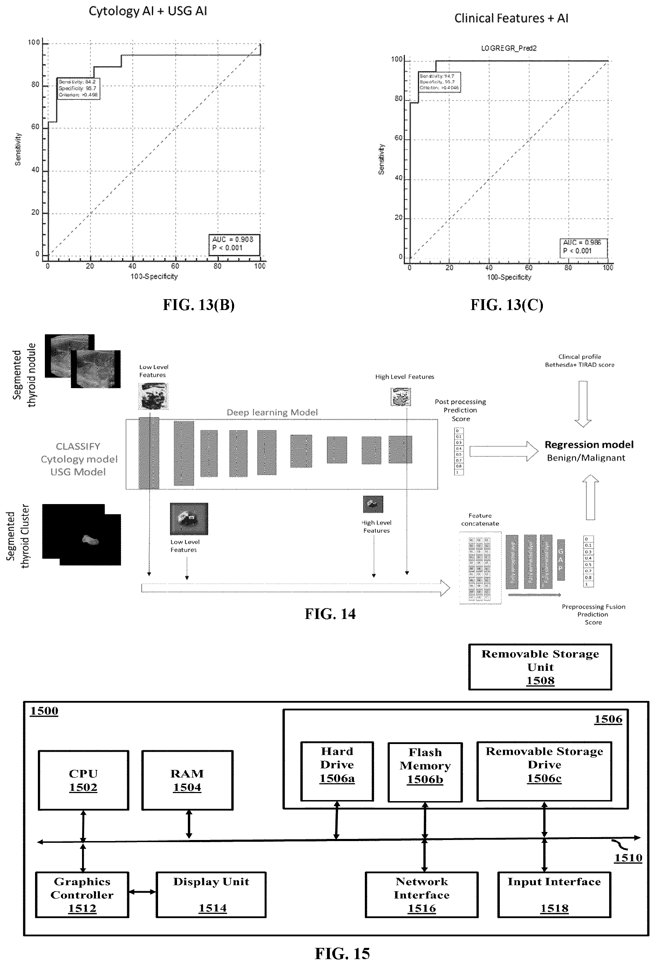

A system for classification of thyroid nodules as benign or malignant, according to aspects of disclosed embodiment is shown in **FIG. 2** **and** **FIG 14****.** The pipeline developed for the development of TTS for the classification is described in detail in the below sections.

### 3. COMPOSITION OF THE DISCLOSED EMBODIMENT

A composition for assessing a condition in a biological sample, wherein the composition comprises of biomarkers WGA, Galectin-3, HBME-1 and MAA; a detection agent that binds to the biomarker expression products; and at least one reagent that allows quantification of biomarkers or biomolecules expression product in a biological sample.

The detection agent that binds to the biomarker expression products is an antibody or a lectin; wherein the reagent that allows quantification of biomarkers is conjugated to fluorescent dyes, wherein the fluorescent dyes are DAPI, TRITC, and FITC; wherein the biomolecules in a biological sample are DNA, protein, antibodies.

The condition is benign or malignancy; wherein biological sample is a thyroid nodule, wherein the biomarkers can differentiate benign from malignant thyroid nodules with sensitivity and specificity, the wherein the biomarkers can predict malignancy or cancer progression.

The biomarkers can differentiate benign from malignant thyroid nodules wherein the sensitivity and specificity of the biomarkers in differentiating benign from malignant thyroid nodules by immunohistochemistry (IHC)) are WGA: 92.5%/83.8%, MAA: 80%/83.8%, Galectin-3: 72.5%/100% and HBME-1: 90%/70.9% respectively, wherein the combination of Galectin-3 and WGA markers illustrated AUC score of 0.96 with 88.3% sensitivity and 90.7% specificity in delineating benign and malignant nodules; wherein the combination of Galectin-3 and WGA markers illustrated AUC score of 0.78 for indeterminate category and AUC score of 1.0 for category I/II.

The composition, wherein the biomarker combination of Galectin-3 and WGA levels in the cytology-based assay illustrated 95% sensitivity and 96% specificity when combined with USG images, in delineating the benign and malignant nodules; wherein the biomarker combination of Galectin-3 and WGA can differentiate cancer and benign nodules of indeterminate nodules with 95% specificity and 80% sensitivity using markers and USG images

### 4. METHODOLOGY OF THE DISCLOSED EMBODIMENT

A method for detection of thyroid cancer in a sample, the method comprising: capturing a set of ultrasonography (USG) images of a thyroid region; capturing a set of molecular cytology images of biological samples of thyroid nodules in the thyroid region; employing a USG-segmentation and classification model to identify a first set of features from the set of USG images, the first set of features characterizing the thyroid nodules in the USG images; employing a molecular cytology-segmentation and classification model to identify a second set of features from the set of molecular cytology images, the second set of pathological features characterizing the thyroid clusters in the molecular cytology images; training a machine learning (ML) model based on a combination of the first set of features and the second set of features; and predicting, based on the ML model, whether a thyroid nodule in the thyroid region is benign or malignant.

### A. MULTIPLEX CYTOLOGY ASSAY

FNAC samples were collected in BD Sure Path (BD Biosciences, USA) and stored at 4⁰C. The Cytospin^{™} 4 (Thermo-Scientific; Cat: A78300003, USA) was used to prepare the cytology smear. The smear was then incubated with RBC lysis buffer (a working solution of 4.5 mL of 0.96pM Ammonium Chloride stock and 0.5mL of 4.25mM TRIS stock; pH adjusted to 7.2) for 3 minutes, followed by washing with PBS and then incubated with TRITC conjugated WGA (1:50 dilution of 1 mg/mL stock solution; EYLabs; Cat: R-2102-5, USA) for 30 minutes. The cells were fixed with 4% paraformaldehyde for 10 minutes. For membrane permeabilization, the cells were incubated with 0.1% Triton-X-100 for 5 minutes, followed by washing with PBS. Alexa Fluor-488 conjugated anti-galectin-3 antibody (1:25 dilution of 0.5 mg/mL; BioLegend; Cat: 125410, India) was added and incubated for 60 minutes and counterstained with nuclear dye, (4', 6-diamidino-2-phenylindole) (DAPI) (1: 1000 dilution of 1mg/mL; Thermo Scientific^{™}; Cat: 62248, Germany).

The images were captured using epifluorescence microscope (Zeiss Axioscope. A1) at 20X objective magnification (Camera: Axiocam ERc5s; resolution: 2560x 1920) in three channels - Tetramethyl rhodamine isothiocyanate (TRITC: EX Bp 546/12, BS FT 560, EM Bp 575-640), Fluorescein Isothiocyanate (FITC: EX Bp 475/40, BD FT 500, EM BP 530/50) and merged channel (DAPI + FITC + TRITC: EX DBP 485/20 + 546/12, BS FT 500+560, EM DBP (515-530) + (580-630)). The data set consists of microscopic images of thyroid FNAC samples stained with two markers, WGA, and Galectin-3 along with DAPI-stained nuclei. The mask was generated in these images manually by the pathologist using the MATLAB labelling library. Images with less than 3 cells were discarded. Images were labelled for thyroid cells (individual cells and clusters), blood cells and artefacts

### B. USG IMAGE ANALYSIS

An expert endocrinologist (>5 years of experience) labelled the thyroid nodules in USG images, and these annotated images were used for segmentation training. The USG images were collected from LOGIQ P9 (GE Healthcare, Milwaukee, USA). A total of 1306 labelled USG images (350 from public data set; KAGGLE dataset) were used for developing the segmentation models. Ninety percent of images were used for training and 10% used for testing after resizing to 256*256*3 pixels.

For the classification model 5555 USG images were taken from public data set, which consist of TIRAD 1 (n=1835), 2 (n=1411), 4 (n=1182) and 5 (n=1127) images. The models were trained for the classification of TIRAD 1/2 and 4/5. The images were in different dimensions and resized into 256*256*3 pixels.

### C. Al SEGMENTED MODEL

An AI segmented model is a different approach in artificial intelligence that breaks down a complex problem into more manageable, specialized components. By dividing the model into distinct segments, each designed to handle specific tasks, the system can achieve higher efficiency and accuracy.

AI segmented models offer significant advantages in scalability and adaptability. As new requirements or tasks emerge, additional segments can be seamlessly integrated into the existing framework. This flexibility is particularly valuable in dynamic environments where the nature of data and tasks can rapidly evolve. Furthermore, segmented models facilitate parallel processing, enabling different segments to operate concurrently. This parallelism boosts the overall processing speed and efficiency making segmented models a powerful tool for handling large-scale, complex problems in various domains such as image processing, speech recognition, and predictive analytics.

### D. IMMUNOHISTOCHEMISTRY

Immunohistochemistry (IHC) is a laboratory technique used to visualize the presence and localization of specific proteins within tissue sections. By employing antibodies that bind to specific antigens, IHC enables researchers and clinicians to detect proteins of interest within a tissue's complex architecture. The process begins with the preparation of tissue samples, which are fixed, sectioned, and placed on slides. Primary antibodies that specifically target the proteins of interest are applied, followed by secondary antibodies that are conjugated to detection molecules, such as enzymes or fluorescent dyes. This enables the visualizations of the proteins when the tissue sections are examined under a microscope. IHC provides crucial insights into the spatial distributions and abundance of proteins, facilitating the study of cellular processes and disease mechanisms.

In clinical setting, IHC plays a vital role in diagnostic pathology. It is frequently used to identify markers of various diseases, including cancer, by highlighting abnormal protein expressions or the presence of pathogens. IHC helps to elucidate the functions and interactions of proteins within tissues. Its ability to provide detailed visual representation of protein localization within the cellular context makes IHC an indispensable tool in both medical diagnostic and biomedical research.

### 5. SYSTEM OF THE DISCLOSED EMBODIMENT

An artificial intelligence (AI) system for the detection of thyroid cancer in a sample, the system comprising: a USG classify model to extract a first set of features from a set of ultrasonography (USG) images captured of a thyroid region, the first set of features characterizing the thyroid nodules in the USG images; a cytology classify model to extract a second set of features from a set of molecular cytology images captures of biological samples of thyroid nodules in the thyroid region, the second set of features characterizing the thyroid clusters in the molecular cytology images; and a machine learning (ML), preprocessing fusion model trained based a combination of the first set of features and the second set of features, wherein the ML mode integrates the preprocessing model with the post-processing fusion model based on the USG-AI score and the cytology-AI score to generate a Thyroid Tumor Score (TTS), thereafter this model is operable to predict whether a thyroid nodule in the thyroid region is benign or malignant (Sensitivity 70%/Specificity 69%).

### 6. EXAMPLE EMBODIMENTS

The disclosed embodiment will be further described in the following examples. It should be understood that these examples are for illustrative purposes only and are not to be construed as limiting this disclosed embodiment in any manner. In addition, the examples may be performed separately, in combination or partially, as will be appreciated by a person skilled in the relevant arts by reading the disclosure herein.

### EXAMPLE 1: SELECTION OF THE MOLECULAR MARKERS USING SYSTEMATIC REVIEW AND META-ANALYSIS

A systematic review-meta-analysis was carried out to identify markers that could significantly differentiate benign from malignant thyroid nodules in FNAC samples. HBME-1 and Gal-3 were identified as the best markers with pooled sensitivity and specificity of 76% and 86% respectively for indeterminate category. Forest plot analysis showed significant association with malignancy (Odds Ratio= 17.37; CI: 9.61-31.38) with significant heterogeneity.

### RESULTS

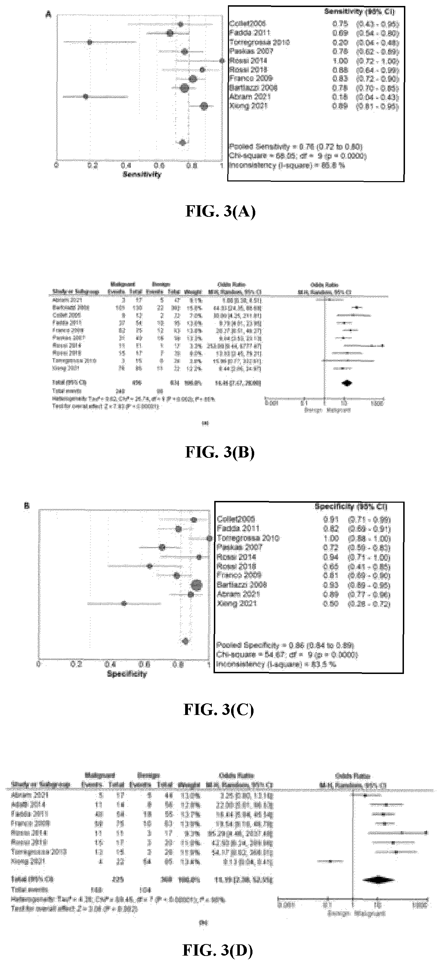

**FIG.s 3(A)-3(D)** illustrate the meta-analysis result of Galectin-3 and HBME in delineating benign and malignant thyroid nodules, according to aspect of disclosed embodiment. Specifically, **FIG. 3(A)** illustrates the Galectin differentiated indeterminate category in meta-analysis. Forest plot analysis of Galectin-3 showed a pooled sensitivity of 76%, while **FIG. 3(B)** illustrates that Galectin-3 differentiated indeterminate into benign and malignant in cytology. Forest plot analysis of Galectin-3 showed pooled specificity of 86%, according to aspect of disclosed embodiment. **FIG. 3(C)** illustrates the Galectin-3 association with malignant thyroid nodules. Forest plot showing Diagnostic Odds Ratio of Galectin-3 (14.46) in delineating indeterminate category of thyroid nodules in FNAC samples, while **FIG. 3(D)** illustrates the HBME-1 association with malignant thyroid nodules- Forest plot showing DOR (11.19) of marker HBME-1 in delineating indeterminate category of thyroid nodules in FNAC sample.

Preliminary studies done on lectins in oral cancer revealed Wheat-Germ-Agglutinin (WGA) and Maackia-Amurensis-Agglutinin (MAA) are potential biomarkers that could differentiate premalignant lesions from cancer

### EXAMPLE 2: VALIDATION OF THE MOLECULAR MARKERS USING IMMUNOHISTOCHEMISTRY

The two markers selected by meta-analysis (Galectin-3 and HBME-1) and lectins (WGA, MAA) were validated in patients with FFPE cohorts (n=120; benign: 43, malignant: 77). Forty-three benign and seventy-seven malignant thyroid nodules were included in this cohort. The malignant nodules were PTC (n= 48), FTC (n= 24), MTC (n= 3), ATC (n=1) and Hurthle cell (n=1) carcinoma. The membrane and cytoplasmic staining of each of the markers, WGA, MAA, Galectin-3 and HBME-1 significantly (p<0.001) delineated the malignant nodules.

### RESULTS

Immunohistochemistry was performed using WGA, Gal-3 and HBME-1 and MAA. The marker staining was significantly (p<0.001) higher in malignant thyroid (n=77) tissues as compared to benign (n=24). The sensitivity and specificity of the markers in differentiating benign from malignant thyroid nodules were WGA: 92.5%/83.8%, MAA: 80%/83.8%, Galectin-3: 72.5%/100% and HBME-1: 90%, 70.9% respectively. The combination Galectin and WGA expression was giving sensitivity and specificity of 85% and 96% respectively.

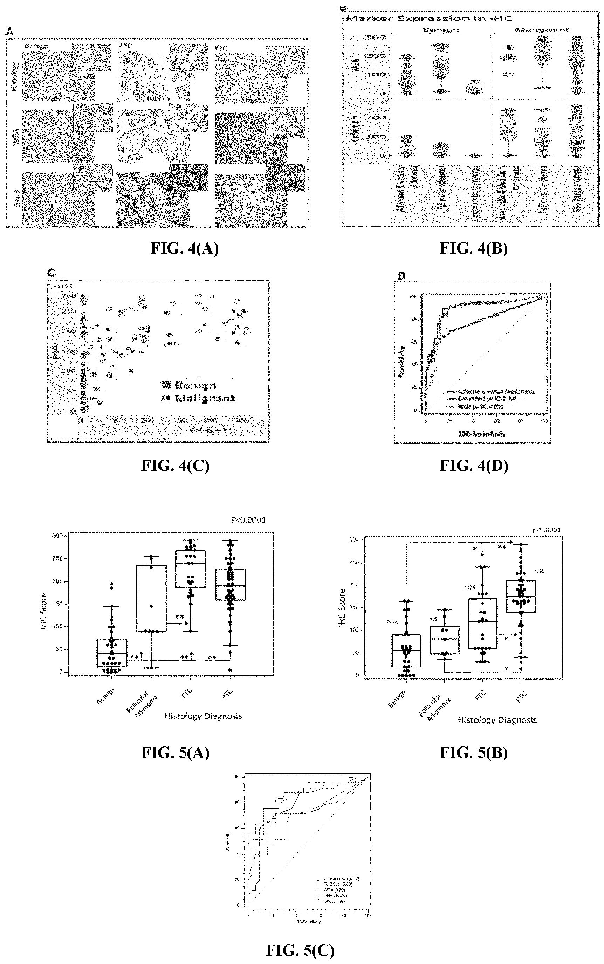

The marker profile significantly increased (in malignant thyroid samples (Papillary Thyroid Cancer: PTC and Follicular Thyroid Cancer: FTC) compared to benign (objective magnification in 10X and 40X) **(****FIG. 4(A)****).** The WGA marker staining is high in follicular cancer compared to Galectin 3 and the levels in the benign cohort **(****FIG. 4(B)****).** The combination of WGA and Galectin 3 differentiated benign and malignant thyroid nodules (ROC AUC: 0.90) compared to single markers **(****FIG.s 4(C)** and **4(D)****).**

**I) GALECTIN-3 AND HBME-1:** The cytoplasmic Galectin-3 expression showed a better accuracy (AUC: 0.83) compared to membrane expression (AUC: 0.80) with moderate sensitivity (71%) and specificity (81.40%). The average cytoplasmic expression of benign and malignant nodules was 63.6 (n=43; SE= 7.23) and 145.67 (n=76; SE= 8.09) respectively **(****FIG 5B****).** The average HBME-1 membrane expression of benign and malignant nodules was 23.48 (n=43; SE= 5.52) and 145.67 (n=77; SE= 7.49) respectively. The membrane expression of HBME-1 showed a low sensitivity (68.83%) but with an 83.72% specificity (AUC: 0.81). Comparatively the cytoplasmic expression of HBME showed an AUC of 0.72 in delineating the malignant lesions. Diagnosis-wise cohort analysis was performed (sample size >5) and showed same trend for Galectin -3 and HBME-1. PTC and FTC showed significantly (p<0.05) high expression compared to all benign nodules, however follicular adenoma (compared to other benign nodules) could not be delineated from follicular carcinoma.

**II) LECTINS:** Membrane expression of WGA showed significantly highest accuracy (AUC= 0.90), similar to the Cohort I, with a sensitivity of 90.91% and specificity of 83.72%. The average membrane expression of benign and malignant nodules was 71.28 (n=43; SE= 10.47) and 199.78 (n=77; SE= 76.70) respectively. The PTC and FTC significantly (p<0.005) delineated benign nodules. The follicular carcinoma significantly delineated follicular adenoma same trend as cohort I **(****FIG. 5(A)****).** The average MAA membrane expression of benign and malignant nodules was 20.93 (n=43; SE = 4.69) and 81.36 (n=77; SE= 7.43) respectively. HBME-1 showed the lowest AUC (0.79). PTC showed a significantly high expression compared to benign and follicular carcinoma, while FTC showed a significantly less expression compared to follicular adenoma/PTC. Multivariate logistic regression analysis showed WGA membrane and Galectin-3 cytoplasmic expression were significant (p<0.01) features using a step-wise method (p<0.05). The combination of these markers showed a significant improvement in AUC (0.96) with a sensitivity 88.3% and specificity of 90.7% in delineating benign and malignant nodules from nodules that are indeterminate (Bethesda III/IV) or TIRAD 3-4 **(****FIG. 5(C)****).**

The correlation of the marker profile with the indeterminate nodules was carried out in patients wherein the Bethesda diagnosis was available (Cohort II; n=102). Bethesda diagnosis included categories I (n=2; 2%), II (28; 28%), III (23, 23%), IV (9, 9%), V (8, 8%) and VI (32, 32%). Pathology correlation indicated that out of the 102 cases, 34 (33%) were benign and 68 (67%) were malignant. The efficacy of individual and combination of markers were evaluated in each Bethesda category.

In Bethesda I/II categories there were 19 benign and 11 malignant nodules. Cytoplasmic Galectin-3 and membrane WGA, HBME -1, MAA significantly delineated benign and malignant nodules of Bethesda I/II category with AUC of 0.95, 0.93, 0.91 and 0.79 respectively. In indeterminate nodules, 15 were benign and 17 were malignant. WGA and Galectin expression delineated indeterminate category significantly with 0.73 and 0.70 respectively. The Bethesda V/VI categories consist of all malignant nodules (n=40). All the four markers showed significantly high expression in Bethesda V/VI nodules compared to other category. The combination of markers (Galectin and WGA) increased the AUC of indeterminate category to 0.78 and category I/II to 1.

**III) CORRELATION OF MARKERS TO TIRAD SCORING: TIRAD correlation was** carried out in ninety samples diagnosed category 1, 2, 3, 4 and 5 were 2, 5, 21, 22 and 40 cases respectively. Benign nodules in each category were 2, 3, 16, 11 and 2 respectively, malignant were 0, 2, 5, 11 and 38 in each category respectively. WGA, Galectin-3, HBME-1 and MAA expression showed significantly high in TIRAD 5 compared to TIRAD 3 and 4. The WGA and Galectin-3 expression accurately delineated benign and malignant thyroid nodules of TIRAD 2 and 5 (AUC>0.96). The TIRAD 3 and 4 category showed AUC of 0.78 and 0.71 respectively in WGA expression. Galectin-3 cytoplasmic expression showed AUC of 0.95 and 0.78 for delineating benign and malignant nodules of TIRAD 3 and 4 respectively. The TIRAD 4 category showed AUC of 0.78. **(****FIG. 5(C)****)**

### EXAMPLE 3: VALIDATION OF COMBINATION OF MOLECULAR MARKERS (WGA AND GAL-3) USING IMMUNOCYTOCHEMISTRY IN PATIENT SAMPLES

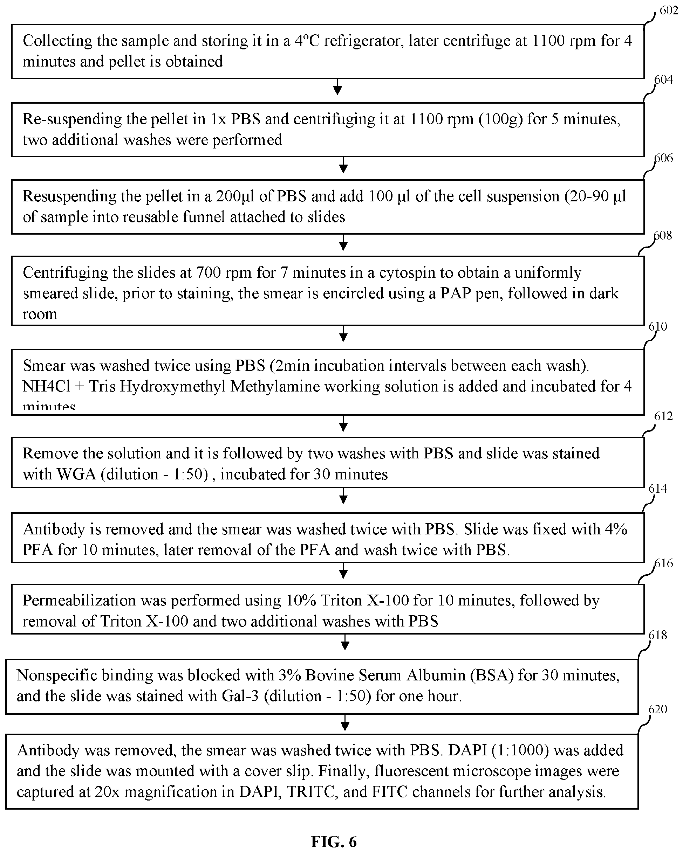

The flow chart for analyzing molecular markers in FNAC samples or Core Needle biopsy is illustrated in **FIG. 6****.**

Thyroid cytology was performed using WGA and Galectin in thyroid fine-needle aspiration cytology (FNAC) samples of thyroid nodules (n=30) as a preliminary study. The procedure for analyzing molecular markers in FNAC samples is as follows: FNAC samples were collected from the Department of Endocrinology and stored in a 4°C refrigerator. The samples were then centrifuged at 1100 rpm for 4 minutes using a non-refrigerating micro-centrifuge **(602).** The resulting pellet was resuspended in 1x PBS and centrifuged at 1100 rpm (100g) for 5 minutes, with two additional washes performed **(604).** The pellet was then re-suspended in a maximum volume of 200µl of (Phosphate buffered solution) PBS, and 100 µl of the cell suspension (20-90µl of sample taken with PBS) was added to a reusable funnel attached to slides **(606).** The slides were centrifuged at 700 rpm for 7 minutes in a cytospin to obtain a uniformly smeared slide. Prior to staining, the smear was encircled using a PAP pen, and all subsequent steps were carried out in a dark chamber **(608).** The smear was washed twice with PBS, with two-minute incubation intervals between each wash. Ammonium chloride (NH4Cl) + TrisHydroxymethyl Methylamine (Tris) working solution was then added and incubated for 4 minutes **(610),** followed by removal of the solution and two additional washes with PBS. The slide was stained with WGA (dilution - 1:50) and incubated for 30 minutes **(612),** after which the antibody was removed and the smear was washed twice with PBS. Subsequently, the slide was fixed with 4% Paraformaldehyde (PFA) for 10 minutes, the PFA was removed, and the smear was washed twice with PBS **(614).** Permeabilization was performed using 10% Triton X-100 for 10 minutes, followed by removal of the Triton X-100 and two additional washes with PBS **(616).** Nonspecific binding was blocked using 3% Bovine Serum Albumin (BSA) for 30 minutes, and the slide was stained with Gal-3 (dilution - 1:50) and incubated for one hour **(618).** The antibody was then removed, and the smear was washed twice with PBS. DAPI (1:1000) was added, and the slide was mounted using a cover slip. Finally, fluorescent microscope images were captured in all three channels (DAPI, TRITC, and FITC) at 20x magnification for further analysis **(620).** Our preliminary analysis showed that a significantly high staining in Bethesda 5&6 compared Bethesda 2 category. Bethesda 3 showed two clusters. The combination of WGA and Galectin 3 staining showed a sensitivity and specificity of 92.3% and 72.7% respectively for delineating malignant lesions (Bethesda VI) from benign (Bethesda II) nodules (Unpublished data).

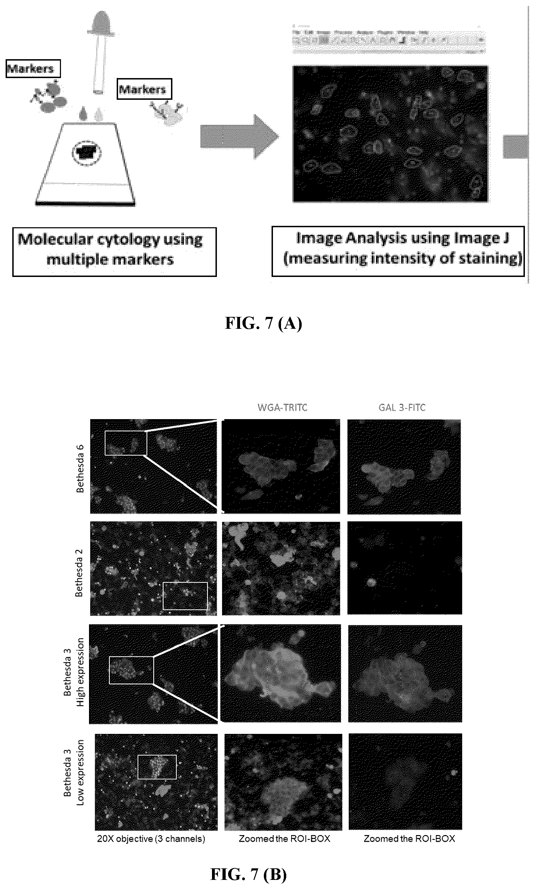

The marker evaluation in cytology is illustrated in **FIG. 7(A)** where the cytology sample stained with markers, and the expression of markers will be evaluated manually using Image. The cell intensity will be measured to assess the efficacy of markers in the delineation of benign and malignant thyroid nodules.

### RESULTS

The multiplex cytology validation of Galectin 3 and WGA is illustrated in **FIG. 7(B)****.** The molecular cytology showed higher levels of WGA and Galectin in thyroid follicular cells in Bethesda 6 (compared to Bethesda 2 objective magnifications in 20X The intensity of cells increased from Bethesda 2 to 6 category (The intensity level is measured using ImageJ).

### EXAMPLE 4: SEGMENTATION AND CLASSIFICATION BASED ON USG IMAGES

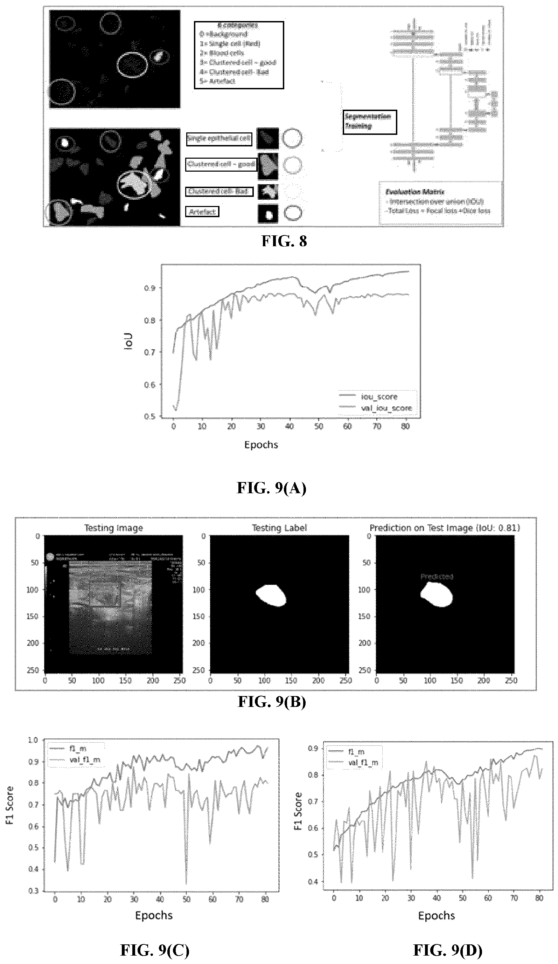

The pathologist will annotate follicular cells (individual/clusters), blood cells, and artefacts of molecular cytology microscopic images. The annotated images will be used for segmentation model training. U-Net architecture will be used for semantic segmentation. The Intersection over Union (IoU) will be used as an evaluation metric for training and testing. Total loss combining focal loss and dice loss will be considered for training. The endpoint will be to obtain an IoU of greater than 0.80 for segmentation of thyroid follicular cells **(****FIG. 8****).**

### RESULTS

**I) USG-SEG Model:** A total of 1306 labelled USG images were used for segmentation of thyroid nodules (Public data set: 350, MSMC: 956). Multiple U-Net models were trained with 1185 images and validated the model in 121 images. Overall nodule, including background) segmentation from USG image was trained using three models, U-Net (training IoU: 0.92; Test: 0.88), Attention U-Net (training IoU: 0.86; Test: 0.87), and Atrous U-Net (training IoU: 0.93; Test: 0.88) **(****FIG. 9(A)****).** Atrous U-Net, achieving the highest nodule IoU of 0.81, was used to predict nodules from MSMC (n= 639) dataset images. **(****FIG. 9(B)****)**
**II) TIRAD-CLASSIFY Model:** For the classification model 5555 USG images were taken from public data set, which consist of TIRAD 1 (n=1835), 2 (n=1411), 4 (n=1182) and 5 (n=1127) images. The Different ResNet models were trained for classification of TIRAD 1/2 and 4/5, and the final model design was validated in the MSMC data set (n: 640). TIRADS classification with ResNet50, ResNet34, and modified ResNet resulted in test F1 scores of 0.83, 0.81, and 0.86 respectively **(****FIG. 9(C)****).** Using Atrous U-Net segmented nodules, the classification of TIRADS 1-2 (n=320) and TIRADS 4-5 (n=319) images achieved training F1 score of 0.93 and a test F1 scores of 0.87.
**III) USG-CLASSIFY Model:** For the development of Thyroid Tumor Score, model is trained for classification of USG images according to Bethesda diagnosis for I/II (n: 363) from Bethesda V/VI (160). The USG image-based model could classify the patients into Bethesda I/II and Bethesda V/VI with a training F1 score of 0.90 and testing F1score of 0.88. **(****FIG. 9(D)****)**

### EXAMPLE 5: AUTOMATION OF USG IMAGE ANALYSIS

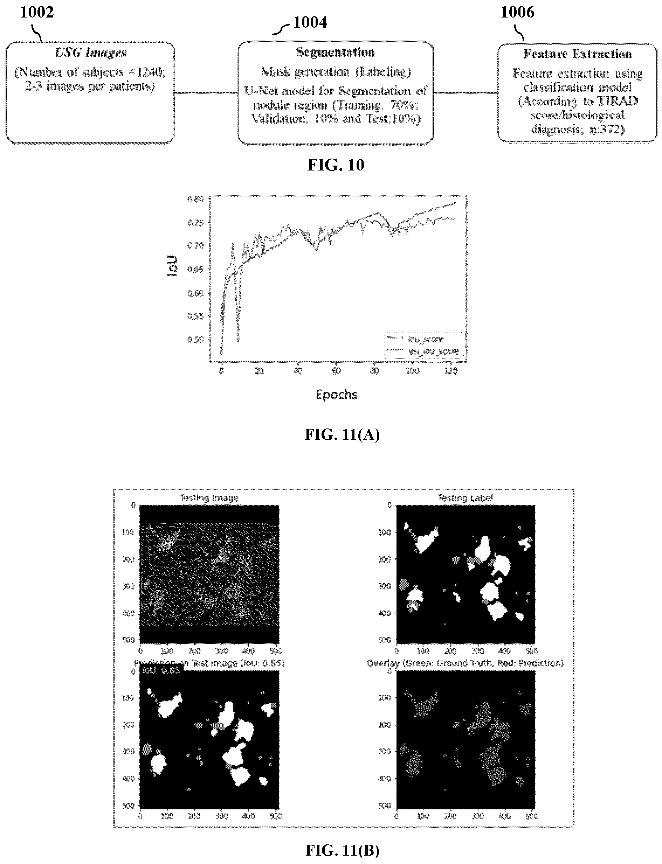

Image quality will be evaluated using Non-Reference Image Quality Evaluator (NIQE). The grey scale USG images will be collected from all centers along with FNAC/histology diagnosis **(1002).** An expert endocrinologist/radiologist (>5 years of experience) will label (mask) the margin of tumor in USG images and these annotated images will be used for segmentation training. USG images will be labeled for malignant/benign nodular areas such as hyperechoic/hypoechoic, irregular margins, TIRAD score or micro-calcification **(****FIG. 10****).**

**AI MODELS:** Decoder-encoder (U-Net) models will be used for segmentation of tumor from the USG images **(1004).** We will use 70% of images for training segmentation algorithms (which have only cytology diagnosis), 20% for validation and 10% for testing. The Intersection over Union (IoU) will be used as evaluation metric for training and testing. Total loss combining focal loss and dice loss will be considered for training. The endpoint will be to obtain an IoU of greater than 0.80 for segmentation of thyroid nodules compared to expert endocrinologist/radiologist.

### EXAMPLE 6: SEGMENTATION AND CLASSIFICATION BASED ON MOLECULAR CYTOLOGY IMAGES

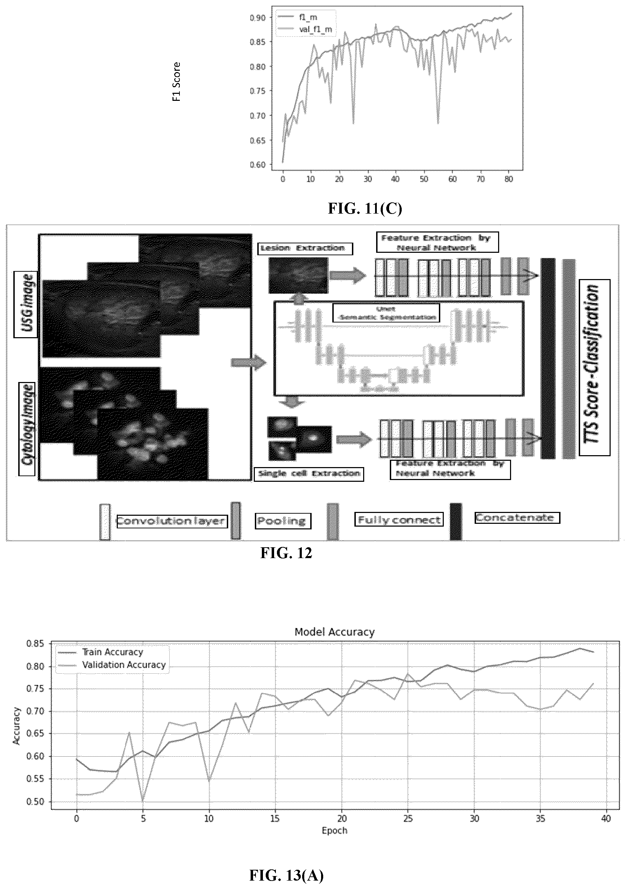

**I) CYTOLOGY-SEG MODEL:** Immunocytology was performed with the combination of molecular markers- WGA and Galectin-3. Multiplexing was completed with 257 samples. U-Net was developed using 495 labelled molecular cytology images. The U-Net model for segmentation of the thyroid cell clusters from the molecular cytology images had a training and test IoUs of 0.78 and 0.76 respectively. The model could segmentation the single cells from the images with a test IoU of 0.54. **(****FIG. 11(A)****)**
**II) CYTOLOGY-CLASSIFY MODEL:** The segmentation model was applied to the patients' ICC data wherein the histological reports were available (number of patients= 50; Benign: 24, Malignant: 26) **(****FIG. 11(B)****).** The clusters were classified using modified ResNet model. For classification of the patients into benign (n=806) and malignant (n=922), the modified ResNet model developed with the segmented clusters from molecular cytology images achieved training and test F1 scores of 0.87 and 0.88 respectively. **(****FIG. 11(C)****)**

### EXAMPLE 7: DEVELOP THYROID TUMOR SCORE (TTS) USING BIOMARKER-BASED CYTOLOGY AND USG IMAGE

The deep learning models used for classification of thyroid USG images will be used for feature extraction from USG images. PCA will be used for reducing the dimension of feature maps. Feature vectors generated for each cytology slide image will be concatenated to form the final feature vector and a machine model will be developed considering histology diagnosis as the reference standard.

For the model development, the data will be split into training (70%), cross validation and test. For the hyper parameter tuning, 3-fold cross-validation of the training data set will be used. The data will be normalized and trained with different classification machine learning models; logistic regression, random forest, KNN, SVM and Xg-Boost.

The diagnostic performance will be calculated based on histology diagnosis as the reference standard. The over-fitting and performance of the model will be assessed by comparing sensitivity, specificity, accuracy, and Area under Curve (AUC) in the training and test data.

The pipeline for development of TTS Score showing Semantic segmentation, feature extraction using neural network (decoder-encoder models) and classification of Benign/malignant nodules (TTS score) are illustrated in **FIG. 12****.**

### RESULTS

We have developed two approaches for combining the USG and molecular cytology models to generating the thyroid tumor score - (i) pre-processing fusion model and (ii) Post processing fusion classification models.
**I) PRE-PROCESSING FUSION MODEL:** The low-level and high-level features of USG and thyroid clustered cell images were extracted using USG classify and cytology classify model respectively. The features are concatenated for developing the fusion model. The model is developed using USG images (n=493) and multiplex cytology clusters (n=1728) using a fully connected neural network. The USG images are augmented for the reaching the same number of images. The training (80% of the dataset), validation (10% of the dataset) and test accuracy was 83%, 78% and 83% respectively **(****FIG. 13(A)****).** The model validated in patient-wise data including benign (n: 19), and malignant (n: 23) patients showed a sensitivity of 69% and specificity of 70% irrespective of all Bethesda categories.
**II) POST-PROCESSING FUSION MODEL:** A Cytology-AI score was generated by calculating a patient-wise average from AI score of each image), which gave a sensitivity and specificity of 79% and 83% respectively. The USG AI score (patient wise average) was giving sensitivity and specificity of 79% and 87% respectively. Logistic regression model was developed combining USG classify and cytology classify model's prediction probability score (average of paired USG and cytology cluster images of patient-wise). The post processing fusion model gave a sensitivity and specificity of 84.2% and 96% (AUC: 0.91) **(****FIG. 13(B)****).** The sensitivity of the model significantly improved after integration of the Bethesda and TIRAD score. The final model gave a sensitivity and specificity of 95% (AUC: 0.99). **(****FIG. 13(C)****)**

### THYROID SCORE FOR INDETERMINATE NODULE:

The post processing fusion model gave a better performance in indeterminate nodules. Out of 42 cases, 48% (n=20; Benign: 15, Malignant: 5) of cases were indeterminate; the model could identify these nodules with a sensitivity of 80% (n=4/5) and specificity of 93% (14/15).

### EXAMPLE 8: PATIENTS RECRUITED FOR Al BASED ANALYSIS

USG-guided FNAC was performed from 351 thyroid nodules (335 participants). Out of the 257 nodules, 5.8% (n=15), 44.4% (n=114), 30.3% (n=78), 4.7% (n=12), 3.5% (n=9), and 11.3% (n=29) were diagnosed as Bethesda category I, II, III, IV, V and VI respectively. USG images were available for 94% (242/257) of nodules. Out of 242 nodules, 1.7% (n=4), 12.4% (n=30), 34.7% (n=84), 33.0% (n=80) and 18.2% (n=44) were of TIRADS 1, 2, 3, 4, and 5 respectively. Seventeen percent (n: 57/335) of the patients, corresponding to 61 nodules have undergone surgery. Out of the 57 patients (61 nodules) - 46% (n: 28) were benign nodules, 47% (n: 29) were Papillary Thyroid Carcinoma, and 7% (n: 4) were Follicular Thyroid Carcinoma. Out of the 57 cases, 41% (n: 25) were of the indeterminate category, with histological diagnosis revealing 44% (11/25) as malignant and 56% (14/25) as benign nodules. Ninety-two percent (23/25) of Bethesda category V/ VI were diagnosed as malignant and 100% of (11/11) of Bethesda I/II were diagnosed as benign nodules.

### 7. ARTIFICIAL INTELLIGENCE (AI) SYSTEM

**FIG. 14** illustrates the final pipeline for classification of benign and malignant thyroid nodule by concatenating the clinical diagnosis (Bethesda/TIRAD), preprocessing fusion model and post-processing fusion model, according to the aspects of the invention.

The pipeline depicts the classification process for benign and malignant thyroid nodules. Thyroid nodules and cell clusters are segmented using an encoder-decoder method from USG images and fluorescent multiplex cytology images, respectively. The segmented nodules are classified according to Bethesda II and Bethesda V/VI categories using a Residual CNN model. Similarly, the segmented cell clusters are classified based on histological diagnosis as either benign or malignant.

Features are extracted from the segmented nodules and cell clusters using the trained Residual CNN model, with low-level features derived from initial CNN blocks and high-level features from final CNN blocks. These features are then concatenated from both USG nodule images and cluster cells.

A preprocessed fusion model is developed using a fully connected neural network. Prediction scores from the classifier models of USG images and cell clusters are utilized to create a post-processing fusion model. The final regression model combines the preprocessed and post-processed fusion models along with clinical diagnosis data (Bethesda/TIRAD scores). The preprocessed fusion model achieved an AUC of 0.71, which increased to 0.91 (sensitivity: 84%, specificity: 96%) when combined with the post-processing scores. Incorporating clinical parameters of TIRAD Bethesda scores further improved the AUC to 0.98, with sensitivity and specificity of 95% and 96%, respectively.

### 8. HARDWARE

**(****FIG. 15****)** is a block diagram illustrating the details of a digital processing system **(1500)** in which various aspects of the present disclosure are operative by execution of appropriate execution modules, firmware or hardware components. Digital processing system **(1500)** may correspond to each of user system: local system or remote and server noted above. Digital processing system may contain one or more processors (such as a central processing unit (CPU) **(1502)),** random access memory (RAM) **(1504),** secondary memory **(1506),** graphics controller (GPU) **(1512),** display unit **(1514),** network interfaces like (WLAN) **(1516),** and input interfaces **(1518).** CPU **(1502)** executes instructions stored in RAM **(1504)** to provide several features of the present disclosure. CPU **(1502)** may contain multiple processing units, with each processing unit potentially being designed for a specific task. Alternatively, CPU **(1502)** may contain only a single general purpose processing unit. RAM **(1504)** may receive instructions from secondary/system memory **(1510).** Graphics controller (GPU) **(1512)** generates display signals (e.g., in RGB format) to primary display unit **(1514)** based on data/instructions received from CPU **(1502).** Primary display unit contains a display screen **(1514)** (e.g., monitor, touchscreen) to display the images defined by the display signals. Input interfaces **(1518)** may correspond to a keyboard, a pointing device (e.g., touch-pad, mouse), a touchscreen, etc. which enable the various inputs to be provided. Network interface **(1516)** provides connectivity to a network (e.g., using Internet Protocol), and may be used to communicate with other connected systems. Network interface **(1516)** may provide such connectivity over a wire (in the case of TCP/IP based communication) or wirelessly (in the case of WIFI, Bluetooth based communication). Secondary memory **(1506)** may contain hard drive (mass storage) **(1506a),** flash memory **(1506b),** and removable storage drive **(1506c).** Secondary memory **(1506)** may store the data (e.g., the specific requests sent, the responses received, etc.) and executable modules, which enable the digital processing system **(1500)** to provide several features in accordance with the present disclosure. Some or all of the data and instructions may be provided on a removable storage unit (SD card) **(1508),** and the data and instructions may be read and provided by removable storage drive **(1506c)** to CPU **(1502).** Floppy drive, magnetic tape drive, CD-ROM drive, DVD Drive, Flash memory, removable memory chip (PCMCIA Card, EPROM) are other examples of such removable storage drive. **(1506c).** Removable storage unit **(1508)** may be implemented using storage format compatible with removable storage drive **(1506c)** such that removable storage drive **(1506c)** can read the data and instructions. Thus, removable storage **(1506c)** unit includes a computer readable storage medium having stored therein executable modules and/or data. However, the computer (or machine, in general) readable storage medium can be in other forms (e.g., non-removable, random access, etc.). CPU **(1502)** may retrieve the executable modules, and execute them to provide various features of the present disclosure described above.

### 9. USES, APPLICATIONS AND BENEFITS OF THE DISCLOSED EMBODIMENT

The combination of molecular markers with radiology brings several benefits to the field of thyroid FNAC and the automation of cytology. This will improve the accuracy of thyroid nodule triaging.

The combination of molecular markers with radiology brings several benefits to the field of thyroid FNAC and the automation of cytology. Here are a few points highlighting their usefulness:

Improved diagnostic accuracy: The markers HBME-1, MAA, Gal-3 and WGA integrated with USG using AI, have demonstrated high sensitivity and specificity in distinguishing between benign and malignant thyroid nodules. Their expression patterns can provide valuable information to aid in the accurate diagnosis of thyroid lesions, reducing the chances of misdiagnosis or unnecessary surgeries. The markers can be applied to core needle biopsy or any other thyroid biopsy that has adequate tissue for the same purpose.

Risk stratification: The use of HBME-1, MAA, Gal-3, and WGA as molecular markers integrated with USG using AI, allows for risk stratification of thyroid nodules. By the intensity of these markers, clinicians can categorize nodules in determinate groups accurately, facilitating appropriate patient management and personalized treatment plans.

Automation of cytology: Incorporating Gal-3 and WGA into automated cytology systems can streamline and standardize the diagnostic process. Automated platforms can efficiently screen and analyze large volumes of FNAC samples, enabling faster turnaround times and reducing the burden on pathologists. The integration of these markers in automated systems enhances the objectivity and reproducibility of results.

Reducing indeterminate cases: Thyroid FNAC occasionally yields indeterminate or inconclusive results, posing challenges for accurate diagnosis. The inclusion of Gal-3 and WGA as molecular markers can help in resolving indeterminate cases by providing additional diagnostic information. This can reduce the need for repeat biopsies or unnecessary surgeries in patients with inconclusive FNAC results.

Enhancing patient care and management: The utilization of Gal-3 and WGA in thyroid FNAC contributes to more precise risk assessment and treatment decision-making. By differentiating between benign and malignant nodules with higher confidence, patients can receive timely and appropriate interventions, leading to improved outcomes and a more personalized approach to thyroid disease management.

Overall, this combination in thyroid FNAC holds promise for enhancing diagnostic accuracy, risk stratification, and the automation of cytology. Incorporating these markers into clinical practice has the potential to optimize patient care and improve the efficiency of thyroid nodule evaluation. The assay reduces the economic burden on the patients, by reducing the need for surgical procedures or genetic testing.

The automation of ultrasound imaging (USG) has several significant implications across various aspects. Here are some key implications of the automation of USG:

Increased Efficiency: Automation simplifies and expedites the ultrasound examination process. Advanced software algorithms and artificial intelligence (AI) tools can automate tasks such as image acquisition, measurement, and analysis, reducing the time required for a complete ultrasound examination. This increased efficiency allows healthcare providers to see more patients, reducing waiting times and improving overall patient care.

Standardization and Consistency: Automation helps in achieving standardized and consistent imaging protocols. Software-driven automation ensures that ultrasound scans are performed using predefined parameters and protocols, minimizing inter-operator variability. This consistency in imaging enhances the accuracy and reliability of diagnostic interpretations, enabling better comparison of images over time.

Enhanced Diagnostic Accuracy: Automated USG tools and AI algorithms that integrate radiology with molecular cytology can assist in detecting and characterizing abnormalities with greater accuracy. These systems can analyze ultrasound images, detect subtle features or abnormalities that might be missed by the human eye, and provide quantitative measurements and objective assessments. The AI-based integration of molecular cytology with USG has the potential to augment diagnostic capabilities and improve accuracy in detecting and diagnosing various conditions.

Integration of Molecular Cytology-AI and USG-AI: The integrated model combining the features from molecular cytology and USG images will increase the accuracy of delineating benign and malignant nodules in thyroid cancers. A further integration with clinical features provided additional accuracy.

Workflow Optimization: Automation streamlines workflow processes in ultrasound departments or clinics. Automated systems can automate image acquisition, data storage, and report generation, reducing manual data entry and paperwork. This optimization of workflow allows healthcare professionals to focus more on patient interaction, interpretation of results, and making informed clinical decisions.

### 10. BEST MODE TO PRACTICE

Conduct extensive validation studies to establish the robustness and reliability of Gal-3 and WGA as molecular markers for thyroid nodule, independently as well as in combination with USG images. Collaborate with research institutions, medical centers, and expert pathologists to generate a significant body of evidence supporting the diagnostic utility and accuracy of these markers.

The markers and the integrated algorithm can be applied to core needle biopsies and other biopsies with adequate tissue to extend the application of the overall assay system

Automated USG systems can contribute to cost savings in multiple ways. By reducing the time required for each examination, healthcare providers can optimize resource allocation and potentially reduce staffing needs. Additionally, automated systems can minimize errors and variability, reducing the need for repeat examinations or additional diagnostic tests.

The combination model of molecular cytology and USG of thyroid nodules can demonstrate a commitment to innovation and cutting-edge healthcare practices. This will be of major advantage in rural centers/ clinics where there is a lack of specialists. The molecular cytology-USG integrated model can be applied in tertiary care health centres to improve the diagnostic accuracy of FNAC/CNB. The assay provides a cost benefit in comparison to the sequencing and other high throughput assays systems currently available.

Merely for illustration, only representative number/type of graph, chart, block, and sub-block diagrams were shown. Many environments often contain many more block and sub-block diagrams or systems and sub-systems, both in number and type, depending on the purpose for which the environment is designed.

Reference throughout this specification to "one embodiment", "an embodiment", or similar language means that a particular feature, structure, or characteristic described in connection with the embodiment is included in at least one embodiment of the disclosed embodiment.

It should be understood that the figures and/or screen shots illustrated in the attachments highlighting the functionality and advantages of the disclosed embodiment are presented for example purposes only. The disclosed embodiment is sufficiently flexible and configurable, such that it may be utilized in ways other than that shown in the accompanying figures.

It should be understood that the examples and embodiments described herein are for illustrative purposes only and that various modifications or changes in light thereof will be suggested to persons skilled in the art and are to be included within the spirit and purview of this application and scope of the appended claims.

## Claims

1. A composition for assessing a condition in a biological sample, wherein the composition comprises of biomarkers WGA, Galectin-3, HBME-1 and MAA; a detection agent that binds to the biomarker products; and at least one reagent that allows quantification of biomarkers or biomolecules expression product in a biological sample.

2. The composition as claimed in claim 1, wherein the condition is benign or malignancy; wherein the biological sample is a thyroid nodule, wherein the biomarkers can differentiate benign and malignant thyroid nodules with sensitivity and specificity, the wherein the biomarkers can classify malignancy in indeterminate nodules.

3. The composition as claimed in claim 1, wherein the biomarkers can differentiate benign from malignant thyroid nodules at tissue level, wherein the sensitivity and specificity of the biomarkers in differentiating benign from malignant thyroid nodules are WGA: 92.5%/83.8%, MAA: 80%/83.8%, Galectin-3: 72.5%/100% and HBME-1: 90%/70.9% respectively, wherein the combination of Galectin-3 and WGA markers illustrated AUC score of 0.96 with 88.3% sensitivity and 90.7% specificity in delineating benign and malignant nodules in tissues; wherein the combination of Galectin-3 and WGA markers illustrated AUC score of 0.78 for indeterminate category and AUC score of 1.0 for category VII.

4. The composition as claimed in claim 1, wherein the biomarker combination of Galectin-3 and WGA levels in the cytology-based assay illustrated 84% sensitivity and 96% specificity when combined with USG images, in delineating the benign and malignant nodules; wherein the biomarker combination of Galectin-3 and WGA can differentiate cancer and benign nodules of indeterminate nodules with 93% specificity and 80% sensitivity using markers and USG images.

5. The composition as claimed in claim 1, wherein the detection agent that binds to the biomarker expression/products is an antibody or a lectin; wherein the reagent that allows quantification of biomarkers expression product is conjugated to fluorescent dyes, wherein the fluorescent dyes are DAPI, TRITC, and FITC; wherein the biomolecules in a biological sample are DNA, protein, antibodies.

6. A method for detection of thyroid cancer in a sample, the method comprising:
capturing a set of ultrasonography (USG) images of a thyroid region;
capturing a set of molecular cytology images of biological samples of thyroid nodules in the thyroid region;
employing a USG-segmentation and classification model to identify a first set of features from the set of USG images, the first set of features characterizing the thyroid nodules in the USG images;
employing a molecular cytology-segmentation and classification model to identify a second set of features from the set of molecular cytology images, the second set of pathological features characterizing the thyroid clusters in the molecular cytology images;
training a machine learning (ML) model based on a combination of the first set of features and
the second set of features; and
predicting, based on the ML model, whether a thyroid nodule in the thyroid region is benign or malignant.

7. The method of claim 6, further comprising:
Generating, using the USG-segmentation and classification model, a USG-AI score for each of the set of USG images;
Generating, using the molecular cytology-segmentation and classification model, a cytology-AI score for each of the set of molecular cytology images; and
providing as inputs to training the ML model, the average of the USG-AI score and cytology-AI score of paired USG and molecular cytology images.

8. The method of claim 7, wherein the ML model is a regression or a non-regression ML model, wherein each of the USG-segmentation and classification model and the molecular cytology-segmentation and classification model is provided as an input to a residual neural network, the method further comprises:
providing as inputs to training the ML model, a clinical diagnosis (Bethesda/TIRAD) of the thyroid region.

9. The method of claim 7, further comprising:
obtaining the biological samples of thyroid nodules from the thyroid region that can be from a Fine Needle Aspiration Cytology (FNAC), Core Needle Biopsy (CNB) or any biopsy with adequate sample;
applying a set of biomarkers to the biological sample; and
capturing images of the stained biological sample to form the set of molecular cytology images.

10. The method of claim 9, wherein the biomarkers are WGA, Galectin-3, HBME-1 and MAA.

11. An artificial intelligence (AI) system for the detection of thyroid cancer in a sample, the system comprising:
a USG-classify model to extract a first set of features from a set of ultrasonography (USG) images captured of a thyroid region, the first set of features characterizing the thyroid nodules in the USG images;
a molecular cytology-classify model to extract a second set of features from a set of molecular cytology images captures of biological samples of thyroid nodules in the thyroid region, the second set of pathology features characterizing the thyroid clusters in the molecular cytology images; and
a machine learning (ML), combining the preprocessing fusion model (Sensitivity 69%/Specificity 70%), trained based a combination of the first set of features and the second set of features, wherein the ML mode integrates the preprocessing model with the post-processing fusion model based on the USG-AI score and the cytology-AI score to generate a Thyroid Tumor Score (TTS), thereafter this model is operable to predict whether a thyroid nodule in the thyroid region is benign or malignant (Sensitivity 95%/Specificity 96%).

12. The AI system of claim 11,
wherein the USG classify model is operable to predict a USG-AI score for each of the set of USG images,
wherein the cytology classify model is operable to predict a cytology-AI score for each of the set of molecular cytology images, and
wherein the post-processing ML model is trained with the average of the USG-AI score and cytology-AI score of paired USG and molecular cytology images (Sensitivity: 84%/Specificity: 95%).

13. The AI system of claim 12, wherein the ML model is a regression or a non-regression ML model, wherein each of the USG-classify model and the cytology-classify model is a residual convoluted neural network (CNN), is integrated with the pre-processing fusion model, wherein the regression ML model is trained with a clinical diagnosis (Bethesda/TIRAD) of the thyroid region.

14. The AI system of claim 13, further comprises:
a first segment model to provide a first segmentation of the set of USG images, wherein the USG classify model is operable based on the first segmentation; and
a second segment model to provide a second segmentation of the set of molecular cytology images, wherein the cytology classify model is operable based on the second segmentation.

15. The AI system of claim 14, wherein the first segment model, and the second segment model are U-Nets (IoU>0.80).
